# EUROPEAN PATENT SPECIFICATION

(11) **EP 0 615 435 B1**
(45) Date of publication and mention of the grant of the patent: **17.09.1997**
(21) Application number: 92923952.3
(22) Date of filing: 01.12.1992
(51) Int. Cl.: A61K 7/48, A61K 31/07

(54) **SKIN CARE COMPOSITION**
HAUTPFLEGEMITTEL
COMPOSITIONS POUR SOINS EPIDERMIQUES

(30) Priority: 03.12.1991 GB 9125712
(43) Date of publication of application: 21.09.1994
(73) Proprietor: SMITHKLINE BEECHAM PLC, Brentford, Middlesex TW8 9EP (GB)
(72) Inventor: CLARKSON, Quinten, Robert, Mark SmithKline Beecham, Weybridge Surrey KT13 0DE (GB); DAVIS, Adrian, Francis SmithKline Beecham, Weybridge Surrey KT13 0DE (GB); GORDON, Jennifer, Jane SmithKline Beecham, Weybridge Surrey KT13 0DE (GB); McGEE, Alan, George SmithKline Beecham, Weybridge Surrey KT13 0DE (GB)
(74) Representative: White, Susan Mary
(86) International application number: GB9202230
(87) International publication number: WO9310754

(56) References cited:
- EP-A- 0 151 953
- EP-A- 0 271 332
- EP-A- 0 272 045
- EP-A- 0 330 496
- WO-A-92/09266
- DE-A- 2 601 489
- US-A- 4 214 000
- US-A- 4 247 547

## Description

This invention relates to skin care compositions containing retinoids for topical application to the skin, in particular to retinoid compositions for the treatment or prophylaxis of acne and for the treatment of skin that is damaged or susceptible to damage by photoaging or exposure to the sun.

Skin care compositions containing retinoid compounds are known for the treatment of acne. The use of topical retinoic acid in the treatment of acne was first described by Kligman A. M. (see US Patent 3,729,568 and Arch. Derm., 99, 469-79, 1969).

More recently, compositions containing retinoid compounds have been described as having a beneficial effect in treating and retarding damage to the skin caused by photoaging and exposure to the sun (UV damage). For example, US Patent 4,603,146 discloses a method for treating sun-damaged human skin topically with retinoic acid.

Early studies with retinoic acid on human skin used doses of retinoic acid up to 1.0% w/w (see Fulton et al., Arch. Derm. 98, 396-399, 1968). At this dosage level, treatment resulted in initial burning, stinging and reddening, scaling and peeling of the skin.

More recent studies have used much lower doses of topical retinoic acid in the range 0.025 to 0.05% w/w. Although these lower dose formulations result in reduced irritation compared with previously used higher doses, significant skin irritation still occurs.

Compositions comprising retinoid compounds formulated in vehicle systems designed to minimise the irritant properties of the retinoid and also maximise the stability of the retinoid in the formulation have been described.

GB 1 476 717 (Johnson & Johnson) discloses gel formulations for topical application for the treatment of acne comprising at least 0.001% by weight of tretinoin (all-trans retinoic acid) and a vehicle system consisting essentially of an organic solvent which is ethanol, isopropanol, propylene glycol or a mixture thereof, an effective amount of a pharmaceutically acceptable antioxidant soluble in the organic solvent and an effective amount of a pharmaceutically acceptable gelling agent solvated in the organic solvent. Examples include a composition comprising tretinoin (0.002% w/w) and hydrocortisone (0.25% w/w) in an ethanol/isopropanol vehicle system.

EP-A-0 266 992 (Ortho) describes compositions containing a mixture of a corticosteroid and a retinoid in a topically administerable, pharmaceutically acceptable carrier for use in treating dermatologic disease. A dose range from about 0.00001% by weight to about 0.3% by weight of retinoid is disclosed but the examples describe only all-trans retinoic acid at a dose level of 0.025% by weight in combination with 0.1% dexamethasone.

EP-A-0 440 398 (Johnson & Johnson) discloses a skin care composition comprising a stable water-in-oil emulsion base including an antioxidant system, a chelating agent and at least one retinoid compound. The retinoid compound is described as being present in a therapeutically effective amount that may range from about 0.0001 to 5.0% by weight of the total composition. The Examples include compositions containing vitamin A alcohol (retinol) at 0.1% w/w, retinyl palmitate at 0.55% w/w, retinyl acetate at 0.34% w/w, vitamin A acid (all-trans retinoic acid) at 0.001% w/w, and 13-cis-retinoic acid at 0.01% w/w.

Many topical formulations containing retinoid compounds, for example commerically available formulations containing retinoic acid in the range 0.025 to 0.5% by weight, have poor topical bioavailability, (see Franz T. J. & Lehman P.A.; J. Toxicol-Col. and Ocular Toxicol. 8(4), 517-524, 1990), that is to say they contain drug concentrations which are very high compared with the fractional amount actually absorbed.

Percutaneous absorption of topically effective drugs is controlled by the concentration of drug in the outer layer of the stratum corneum which is in turn determined by the concentration of drug in the selected vehicle system and the properties of the solvents making up the selected vehicle system. As drug concentration is reduced, percutaneous absorption decreases due to depletion. Thus if compositions containing retinoids are to be formulated at low dosage levels to avoid wastage and local overdosing due to poor bioavailability, optimised vehicle systems are required.

The solubility of active substances in solvent systems is important in relation to the design of topical drug delivery systems. It has been shown that the degree of saturation of an active substance, for example a drug, in the solvent system or vehicle is a determining factor in controlling release of the active substance.

Coldman et al.; J. Pharm. Sci., 58, 1098-1102, 1969, demonstrated that percutaneous absorption could be enhanced by over-saturating a drug solution to a supersaturated level. A supersaturated state is generated when the concentration of a solute, for example a drug, in a given solvent system exceeds the saturated solubility of the solute in that system.

Coldman prepared a solution of a drug in a mixture of a volatile and a non-volatile solvent and applied it to the surface of a sample of human skin. The volatile solvent evaporated leaving the drug in solution in the non-volatile solvent at a concentration in excess of its saturated solubility in that solvent, thereby creating a supersaturated solution in situ on the skin surface.

European Patent No. 0 151 953 (Beecham Group) describes a pharmaceutical composition for generating a drug solution in a supersaturated state which is not reliant on the prior evaporation of a volatile solvent.

The composition comprises two distinct but miscible liquid phases, at least one of which contains a drug dissolved therein. The composition of the phases is such that each has a different lipophilicity (or polarity) and each confers a different saturated solubility on the drug. The composition of the liquid phases and the concentration of drug in one or both phases is such that on admixture of the two phases, the total drug concentration in the mixture thus formed is greater than the concentration of drug which a mixture of the same composition can accommodate as a saturated solution. On mixing the two liquid phases, the resulting mixture is therefore supersaturated with respect to the drug. EP 0 151 953 lists the anti-acne drug retinoic acid as a suitable drug for use in the compositions described therein.

International Patent Application Publication No. WO 92/09266 (Beecham Group; Publication Date 11 June, 1992) discloses further two-phase compositions for topical application wherein one of the liquid phases comprises water. The Examples of WO 92/09266 include formulations in which one of the liquid phases of the two-phase compositions comprises either retinyl propionate (0.01% w/w) or retinoic acid (0.02% w/w) which on mixing of the two phases give rise to supersaturated compositions in which the concentration of retinyl propionate and retinoic acid are 0.002% and 0.0025% by weight respectively.

It is notable that whilst a number of prior art references describe low dose retinoid compositions comprising a combination of volatile and non-volatile solvents, saturated drug solubility and hence percutaneous absorption is not optimised, and supersaturated solutions of the retinoid compounds are not generated on evaporation of the volatile solvents.

Whilst the need for low-dose retinoic acid compositions is recognised in the art in order to reduce irritancy, it has not heretofore been possible to deliver retinoic acid to the skin from a composition in which the retinoic acid concentration is sufficient to promote percutaneous absorption to achieve the desired therapeutic effect whilst avoiding or at least mitigating local irritation. The minimum concentration of retinoic acid in a commercially available anti-acne treatment is 0.025% w/w for a cream formulation and 0.01% w/w for a gel formulation.

It has now been found that a composition containing a low concentration of a retinoid compound, formulated in a vehicle system to deliver a low dosage but supersaturated solution of the retinoid compound to the skin, provides an effective delivery system for enhancing percutaneous absorption to levels achieved with commercially available preparations. Furthermore use of such low-dose retinoid compositions reduces the potential for local overdosing and irritation at permeable skin sites, and renders them particularly suitable for both topical treatment and maintenance therapy over an extended period of time.

According to the present invention there is provided a composition for topical application to the skin comprising a retinoid compound and a vehicle system for the retinoid compound, characterised in that the concentration of the retinoid compound is in the range 0.0001 to 0.004% by weight of the composition and the vehicle system is formulated to deliver a supersaturated solution of the retinoid compound to the skin surface.

As used herein, the term retinoid compound includes all-trans retinoic acid (tretinoin), and 13-cis retinoic acid (isotretinoin) and derivatives thereof including esters and amides; etretinate; retinal; retinol (vitamin A) and deriatives thereof including retinyl ethers and retinyl esters such as retinyl propionate. The term retinoid compound also includes, where applicable, salts, for example alkali metal salts and alkaline earth metal salts, and solvates including hydrates.

The concentration of the retinoid compound in a composition of the invention lies in the range 0.0001 to 0.004% by weight of the total composition, suitably in range 0.00025 to 0.003%, preferably in the range 0.0005 to 0.0025% or 0.0005 to 0.002% by weight, for example 0.00025, 0.0005, 0.00125, 0.002 or 0.0025% by weight.

Preferred retinoid compounds for use in compositions of the present invention for the treatment of acne include all-trans retinoic acid (tretinoin) and 13-cis retinoic acid (isotretinoin). Preferred retinoid compounds for the treatment of photoaging or sun-damaged skin include all-trans retinoic acid and retinyl propionate.

A suitable concentration of tretinoin in a composition for acne treatment lies in the range 0.0001 to 0.003% w/w, preferably 0.00025 to 0.0025% by weight, or 0.0005 to 0.002% by weight, for example 0.00025, 0.0005, 0.00125, 0.002 or 0.0025% by weight.

A suitable concentration of tretinoin in a composition for the treatment of photoaged or sun-damaged skin lies in the range 0.0001 to 0.004%, preferably 0.0005 to 0.003% and more preferably 0.001 to 0.0025% by weight or 0.001 to 0.002% by weight, for example 0.0005, 0.00125, 0.002 or 0.0025% by weight.

Compositions of the invention may be formulated as either single-phase or two-phase compositions. Two-phase compositions of the invention are suitably formulated as described in EP 0 151 953 and preferably as described in WO 92/09266.

Due to the inefficiency of percutaneous absorption, highly supersaturated systems can be of great benefit. The rate of drug penetration in situ will depend largely on the degree of supersaturation, vis the ratio of supersaturated drug concentration to saturated drug concentration. A degree of supersaturation in excess of 1 is considered useful, and values from 2, for relatively slow penetration, to 10, for rapid penetration, are preferred. By means of the present invention very high degrees of supersaturation may be both obtained and moreover maintained over a substantial time period.

Depending upon the formulation type used, single-phase or two-phase, supersaturation is suitably formed or maintained by loss of volatile vehicle components. However, independent of loss of vehicle components and the subsequent increase in the concentration of retinoid compound in the residual phase composition so formed, formulations of the present invention when applied at a typical total product loading, for example between 2.5 to 10 mg/cm², will result in a retinoid compound loading which is very much lower than that contained in the same total product loading of retinoid compositions currently available in the art.

In one aspect of the invention there is provided a two-phase composition for topical application, wherein the two phases are intended to be mixed together on or immediately prior to application, comprising: a first liquid phase containing a retinoid compound dissolved therein and comprising a topically acceptable solubiliser; and a second liquid phase, physically and/or chemically different from the first phase but miscible therewith on admixture, optionally containing the same retinoid compound dissolved therein and comprising a topically acceptable carrier; the composition of the first and second liquid phases being such that each has a different lipophilicity and each confers a different saturated solubility on the retinoid compound; the concentration of retinoid compound in each phase in which it is present and the composition of each of the first and second liquid phases being such that, on admixture of the phases, the total retinoid compound concentration in the mixture thus formed is greater than the saturated retinoid compound concentration in the same mixture, whereby the said mixture is supersaturated with the retinoid compound; characterised in that the retinoid compound concentration in the mixture is in the range 0.0001 to 0.004% by weight of the total composition.

In the context of the present invention, the term liquid is used to denote materials of varying consistency ranging from lotions to viscous materials, in particular creams and gels. Preferably, compositions of the invention, whether single-phase or two-phase, are formulated to provide hydrophilic cosolvent gels.

It will be appreciated that two-phase compositions are not limited with respect to the physical nature of the product obtained on mixing the two liquid phases, provided that the first and second liquid phases are miscible.

In a two-phase composition of the invention, the second liquid phase need not contain any retinoid compound, provided that the product obtained on admixture of the two phases is supersaturated with respect to the retinoid compound. Each phase may contain one or more retinoid compounds in amounts such that the resultant product mixture is supersaturated in one or more retinoid compounds.

Suitably, a two-phase composition of the invention has a first liquid phase which is either subsaturated or saturated with retinoid compound. The use of saturated solutions maximises the degree of supersaturation which a two-phase composition may generate whereas for ease of manufacture it may be advantageous to use a subsaturated concentration of the retinoid compound. Preferably, a composition of the invention has a first liquid phase which is either sub-saturated or saturated with retinoid compound and a second liquid phase which contains no retinoid compound. The degree of saturation, and hence the rate of drug release from the resulting supersaturated drug preparation after mixing, can be readily predicted from the saturated solubility curve for a given solubiliser/carrier system.

In a two-phase composition of the invention, the relative proportion by weight of the first liquid phase to the second liquid phase is advantageously from 1:1 to 1:12, preferably from 1:2 to 1:8.

As used herein with respect to any composition of the invention, the term solubiliser denotes a liquid in which the retinoid compound has a higher saturated solubility than in an associated carrier.

Analogously, the term carrier denotes a liquid in which the retinoid compound has a lower saturated solubility than in an associated solubiser.

Suitably a solubiliser is a liquid in which the retinoid compound is readily soluble whilst a carrier is a liquid in which the retinoid compound has poor solubility.

In a two-phase composition of the invention which is formulated to provide a hydrophilic gel, the topically acceptable carrier of the second liquid phase, as hereinbefore defined, suitably comprises water. Solvent evaporation, in particular evaporation of water which takes place after mixing together of the two liquid phases and application to the skin surface, can have the effect of increasing the saturated solubility of the retinoid compound in the residual composition so formed. An increase in retinoid compound saturated solubility is reflected in a reduction of the degree of saturation of the supersaturated solution. In a preferred two-phase composition of the invention, the tendency for the degree of saturation to decline due to solvent evaporation over a period of time is counteracted by the use of a second liquid phase wherein the topically acceptable carrier comprises a first component which is water and a second component which has a lipophilicity intermediate between that of water and the solubiliser of the first liquid phase.

Since water is a necessary component of the topically acceptable carrier of the second liquid phase of these preferred compositions, it will be readily appreciated that topically acceptable solubilisers suitable for use in such two-phase compositions of the present invention are generally more lipophilic or less-polar liquids. The first liquid phase may comprise more than one such liquid.

Examples of suitable solubilisers include propylene glycol, 1,3-propylene diol, polyethylene glycol, polypropylene glycol, ethanol, propanol, acetone, dimethylisosorbide, dimethylsulphoxide, benzyl alcohol, and other glycol, ether and ester solvents of similar polarity.

Preferred solubilisers are propylene glycol, polyethylene glycol and ethanol.

In a preferred two-phase composition as hereinbefore described, the second component of the topically acceptable carrier of the second liquid phase is a liquid miscible with water, suitably having a lipophilicity closer to that of water than that of solubiliser. Favourably the second component is not volatile at ambient, and particularly at body temperature.

Suitable liquids include glycerol and propylene glycol. A preferred liquid is glycerol.

This second component may comprise up to 50% by weight of the topically acceptable carrier, suitably from 5 to 40% by weight and preferably from 10 to 25% by weight.

In another preferred two-phase composition of the invention, the solubiliser of the first liquid phase may comprise a first component which is non-volatile and a second component which is relatively more volatile at ambient, and particularly at body temperature. Favourably the second more volatile component has comparable volatility to water. Suitable more volatile components include ethanol, isopropanol and acetone. A preferred more volatile component is ethanol. Suitably, a relatively more volatile second component may comprise up to 50% by weight of the first liquid phase.

The invention also encompasses two-phase compositions in which a relatively more volatile solubiliser, for example ethanol, is present in the second liquid phase. The second liquid phase suitably comprises up to 20% by weight of such relatively more volatile solubiliser, for example from 4% to 20% of such relatively more volatile solubiliser.

The incorporation of a more volatile solubiliser component with comparable volatility to water in one or both liquid phases, further counteracts the tendency for the degree of supersaturation in the supersaturated preparation, generated on mixing, to decline. Co-evaporation of this more volatile component with water further stabilises the lipophilicity (or polarity) of the resulting mixture and hence the drug saturated solubility.

Thus, a preferred two-phase composition of the invention containing 0.0001 to 0.004% w/w retinoic acid is formulated to provide an essentially aqueous-based lotion or gel in a 1.5 to 10-fold supersaturated state. The composition suitably comprises greater than 60% w/w water and optionally up to 20% w/w ethanol, propylene glycol and glycerol making up the remainder of the cosolvent system which may also contain a further solvent having a lipophilicity between that of propylene glycol and glycerol.

Where retinoid compositions of the invention are formulated as single phase compositions, the retinoid compound is present at a concentration in the range 0.0001 to 0.004% by weight of the composition which suitably includes a vehicle system comprising a mixture of a volatile solvent and a non-volatile solvent, as first described by Coldman et al., (J. Pharm. Sci. 58, 1098-1102, 1969). The vehicle system is chosen to generate a residual composition in which the concentration of the retinoid compound exceeds its saturated solubility and which accordingly delivers a supersaturated solution of the retinoid compound to the skin after evaporation of the volatile solvent.

Suitable volatile solvents for the vehicle system of a single phase composition of the invention include ethanol, and mixtures of ethanol and water. Suitable non-volatile solvents include propylene glycol, polyethylene glycol, propylene carbonate and glycerol and mixtures thereof. A particularly preferred single-phase composition for retinoic acid comprises a vehicle system containing ethanol, water, propylene glycol and glycerol in proportions which take into account both their relative volatilities and the saturated solubility of retinoic acid in each solvent.

Compositions of the invention may also contain an antinucleating agent.

The antinucleating agent used in two-phase compositions according to the invention may be present in either or both of the said first and second liquid phases of the composition. Advantageously, it is present in at least the second phase and it may additionally be present in the first phase. In any event, when the two phases are mixed to provide a superstaturated solution, the antinucleating agent will, of course, be present in the resulting solution.

The antinucleating agent may be present in an amount of up to 10% by weight, suitably in an amount of up to 5.0% by weight, advantageously from 0.01 to 2.0% by weight, and preferably from 0.1 to 1.0% by weight, based on the total weight of the composition.

The antinucleating agent should be soluble or dispersible in the phase or phases in which it is present and, of course, for a two-phase composition in the resulting mixed solution.

Examples of suitable antinucleating agents are hydroxyalkylcelluloses, such as hydroxypropylmethylcellulose and hydroxypropylcellulose, polyvinylpyrrolidone, polyacrylic acid, and derivatives thereof. A mixture of two or more different antinucleating agents may be used. In the event that an antinucleating agent is included in each of the first and second liquid phases of a two-phase composition, the same or different antinucleating agents may be included in each phase.

The choice of suitable antinucleating agent will depend both on the particular retinoid compound and the choice of solvent materials, but suitable anti-nucleating agents can readily be selected by simple experiment. This may be done, for example, by preparing samples of the desired supersaturated drug solution; adding a selection of antinucleating agents (in say 1% by weight concentration), one to each sample; allowing the samples to stand for say 2 hours; and noting which solutions have remained clear.

Compositions of the invention including each of the first and second liquid phases of a two-phase composition may be thickened with a suitable thickening or gelling agent of either natural or synthetic origin. Examples of thickening and gelling agents are natural gums, tragacanth, carageen, pectin, agar, alginic acid, cellulose ethers and esters, xanthan gum, guar and locust bean gum, bentonite (a colloidal hydrated aluminium silicate), veegum (colloidal magnesium aluminium silicate), laponite (a synthetic hectorite), polyvinyl alcohol, Pluronics (a Tradename), Aerosil (a Tradename colloidal silica), and Carbopol (a Tradename). Where present, a thickening or gelling agent suitably comprises 0.25 to 1.0 % w/w of the composition.

Certain thickening agents may require the addition of an adjunct which serves to activate the thickening mechanism. For example, amines such as triethanolamine or tromethamine (Tris amino) are commonly used in conjunction with Carbopol suspensions. As an alternative, sodium hydroxide may be used, and is particularly suitable for activating aqueous Carbopol suspensions.

Preservatives including anti-oxidants and UV absorbers, and other adjuvants may also be added. It is notable that all-trans retinoic acid (tretinoin) is highly sensitive to UV radiation and oxidative degradation and will readily isomerise to the corresponding cis-isomer (isotretinoin). It is accordingly recommended when handling and storing tretinoin compositions to avoid exposure to UV light. Suitable antioxidants include butylated hydroxyanisole (BHA), butylated hydroxytoluene (BHT) and tertiary butyl hydroquinone (TBHQ). An antioxidant suitably comprises from 0.001 to 0.5% w/w of the composition. Suitable sunscreens include benzophenone and derivatives thereof.

Compositions of the invention may be prepared by processes well known in the art of pharmaceutical formulation, for example by admixture, using appropriate equipment and techniques, of the components present in either a single-phase composition or, where the composition is formulated as a two-phase composition, in each of the first and second liquid phases.

Two-phase compositions of the invention may be packaged into a twin compartment pack ready for topical application by the user or patient. The user or patient would normally apply the two phases simultaneously to the treatment area and then mix the phases together in situ to create the supersaturated drug system.

The two phases may also be mixed in the pack by breaking a membrane or seal separating the first and second phases, thus creating a supersaturated solution in the pack, prior to application. Suitable packs for such purposes are commercially available.

In a further aspect of the invention there is provided a method for topical treatment of the human or animal body which comprises applying thereto an effective amount of a single-phase or a two-phase pharmaceutical composition according to the invention.

The invention also provides the use of a single-phase or two-phase pharmaceutical composition as hereinbefore defined for the manufacture of a medicament for topical application to the skin for the treatment and/or prophylaxis of acne or damage caused by ageing or exposure to UV radiation.

The following Examples illustrate the invention. Examples 1 to 11 provide two-phase formulations which on mixing the two phases generate supersaturated solutions. In each of Examples 1 to 10 a supersaturated solution is formed by mixing one part of the first phase with seven parts of the second phase. In Example 11, a supersaturated solution is formed by mixing one part of the first phase with four parts of the second phase. Examples 12 to 15 are single-phase compositions of the invention which generate a supersaturated solution of retinoic acid by evaporation of the volatile solvents on the skin surface.

In addition to the constituents described in the Examples, the compositions may each contain, as appropriate and where not already indicated, adjuvants such as antinucleating agents, for example HPC, HPMC and PVP; antioxidants, for example butylated hydroxyanisole; preservatives, for example phenoxetol; gelling or thickening agents, for example Carbopol 980 with a suitable neutralising agent such as Tris amino for a non-aqueous phase or sodium hydroxide for an aqueous phase; and UV absorbers, for example benzophenone-3.

The following abbreviations are used:
- PEG: : polyethylene glycol
- PVP: : polyvinylpyrrolidone
- HPMC: : hydroxypropylmethylcellulose
- HPC: : hydroxypropylcellulose

### Example 1

| | | % w/w |
|---|---|---|
| First Phase: | retinoic acid | 0.004 |
| | propylene glycol | 49.998 |
| | ethanol | 49.998 |

| | | % w/w |
|---|---|---|
| Second Phase: | glycerol | 12.00 |
| | water | 87.00 |
| | HPMC | 1.00 |

Concentration of retinoic acid on mixing = 0.0005% w/w
   (Degree of supersaturation = 5.88)
Concentration of retinoic acid in residual composition ≅ 0.003% w/w
   (Degree of supersaturation = 6.43)

### Example 2

| | | % w/w |
|---|---|---|
| First Phase: | retinoic acid | 0.004 |
| | propylene glycol | 99.996 |

| | | % w/w |
|---|---|---|
| Second Phase: | glycerol | 19.00 |
| | water | 75.00 |
| | ethanol | 5.00 |
| | HPMC | 1.00 |

Concentration of retinoic acid on mixing = 0.0005% w/w
   (Degree of supersaturation = 3.44)
Concentration of retinoic acid in residual composition = 0.0016% w/w
   (Degree of supersaturation = 3.80)

### Example 3

| | | % w/w |
|---|---|---|
| First Phase: | retinoic acid | 0.004 |
| | propylene glycol | 99.996 |

| | | % w/w |
|---|---|---|
| Second Phase: | glycerol | 25.00 |
| | water | 74.00 |
| | HPMC | 1.00 |

Concentration of retinoic acid on mixing = 0.0005% w/w
   (Degree of supersaturation = 5.0)
Concentration of retinoic acid in residual composition = 0.0013% w/w
   (Degree of supersaturation = 3.15)

### Example 4

| | | % w/w |
|---|---|---|
| First Phase: | retinoic acid | 0.004 |
| | propylene glycol | 69.996 |
| | glycerol | 30.000 |

| | | % w/w |
|---|---|---|
| Second Phase: | glycerol | 25.00 |
| | water | 74.00 |
| | HPMC | 1.00 |

Concentration of retinoic acid on mixing = 0.0005% w/w
   (Degree of supersaturation = 6.0)
Concentration of retinoic acid in residual composition = 0.0013% w/w
   (Degree of supersaturation = 3.7)

### Example 5

| | | % w/w |
|---|---|---|
| First Phase: | retinoic acid | 0.004 |
| | propylene glycol | 74.997 |
| | glycerol | 24.999 |

| | | % w/w |
|---|---|---|
| Second Phase: | glycerol | 9.50 |
| | water | 84.5 |
| | ethanol | 5.00 |
| | antinucleant | 1.0 |

Concentration of retinoic acid on mixing = 0.0005% w/w
   (Degree of supersaturation = 5.26)
Concentration of retinoic acid in residual composition = 0.0022% w/w
   (Degree of supersaturation = 6.83)

### Example 6

| | | % w/w |
|---|---|---|
| First Phase: | retinoic acid | 0.02 |
| | propylene glycol | 99.98 |

| | | % w/w |
|---|---|---|
| Second Phase: | glycerol | 6.00 |
| | propylene glycol | 4.00 |
| | water | 74.00 |
| | ethanol | 16.00 |

Concentration of retinoic acid on mixing = 0.0025% w/w
   (Degree of supersaturation = 6.00)
Concentration of retinoic acid in residual composition = 0.01% w/w
   (Degree of supersaturation = 6.00)

### Example 7

| | | % w/w |
|---|---|---|
| First Phase: | retinoic acid | 0.02 |
| | propylene glycol | 99.98 |

| | | % w/w |
|---|---|---|
| Second Phase: | glycerol | 15.30 |
| | propylene glycol | 2.7 |
| | water | 71.00 |
| | ethanol | 10.00 |
| | antinucleant | 1.00 |

Concentration of retinoic acid on mixing = 0.0025% w/w
   (Degree of supersaturation = 10.0)
Concentration of retinoic acid in residual composition = 0.008% w/w
   (Degree of supersaturation = 10.3)

### Example 8

| | | % w/w |
|---|---|---|
| First Phase: | retinoic acid | 0.02 |
| | propylene glycol | 99.98 |

| | | % w/w |
|---|---|---|
| Second Phase: | glycerol | 14.25 |
| | water | 79.75 |
| | ethanol | 5.0 |
| | antinucleant | 1.0 |

Concentration of retinoic acid on mixing = 0.0025% w/w
   (Degree of supersaturation = 20)
Concentration of retinoic acid in residual composition = 0.01% w/w
   (Degree of supersaturation = 22)

### Example 9

| | | % w/w |
|---|---|---|
| First Phase: | retinoic acid | 0.01 |
| | propylene glycol | 99.99 |

| | | % w/w |
|---|---|---|
| Second Phase: | glycerol | 12.825 |
| | propylene glycol | 1.425 |
| | water | 79.75 |
| | ethanol | 5.0 |
| | antinucleant | 1.00 |

Concentration of retinoic acid on mixing = 0.00125% w/w
   (Degree of supersaturation = 8.9)
Concentration of retinoic acid in residual composition =0.0046% w/w
   (Degree of supersaturation = 7.8)

### Example 10

| | | % w/w |
|---|---|---|
| First Phase: | retinoic acid | 0.01 |
| | propylene glycol | 99.99 |

| | | % w/w |
|---|---|---|
| Second Phase: | glycerol | 6.00 |
| | propylene glycol | 4.00 |
| | water | 73.00 |
| | ethanol | 16.0 |
| | antinucleant | 1.0 |

Concentration of retinoic acid on mixing = 0.00125% w/w
   (Degree of supersaturation = 3)
Concentration of retinoic acid in residual composition = 0.005% w/w
   (Degree of supersaturation = 3)

### Example 11

| | | % w/w |
|---|---|---|
| First Phase: | retinyl propionate | 0.01 |
| | PEG 400 | 99.99 |

| | | % w/w |
|---|---|---|
| Second Phase: | PEG 400 | 25.00 |
| | glycerol | 75.00 |

Concentration of retinoic acid on mixing = 0.002% w/w
   (Degree of supersaturation ≅ 5)

### Example 12

| | | % w/w |
|---|---|---|
| Single-Phase system: | retinoic acid | 0.0005 |
| | glycerol | 18.0 |
| | propylene glycol | 2.0 |
| | klucel L | 0.005 |
| | carbopol 980 | 0.50 |
| | Tris amino | 0.25 |
| | BHA | 0.25 |
| | ethanol | 55.00 |
| | water | qs to 100 |

Concentration of retinoic acid in residual composition = 0.0025% w/w
   (Degree of supersaturation = 5.0)

### Example 13

| | | % w/w |
|---|---|---|
| Single-Phase system: | retinoic acid | 0.00025 |
| | glycerol | 9.00 |
| | propylene glycol | 1.00 |
| | klucel L | 0.0025 |
| | carbopol 980 | 0.50 |
| | Tris amino | 0.25 |
| | BHA | 0.20 |
| | ethanol | 62.00 |
| | water | qs to 100 |

Concentration of retinoic acid in residual composition = 0.0025% w/w
   (Degree of supersaturation = 5.0)

### Example 14

| | | % w/w |
|---|---|---|
| Single-Phase system: | retinoic acid | 0.0025 |
| | glycerol | 45.00 |
| | propylene glycol | 5.00 |
| | ethanol | qs to 100 |

Concentration of retinoic acid in residual composition = 0.005% w/w
   (Degree of supersaturation = 10.0)

### Example 15

| | | % w/w |
|---|---|---|
| Single-Phase system: | retinoic acid | 0.00125 |
| | glycerol | 45.00 |
| | propylene glycol | 5.00 |
| | ethanol | qs to 100 |

Concentration of retinoic acid in residual composition = 0.0025% w/w
   (Degree of supersaturation = 5.0)

### Example 16

### Comparison of retinoic acid loading in compositions applied to the skin

| Retinoic Acid Concentration (w/w) | Total Product Loading (mg/cm²) | Retinoic Acid Loading (ng/cm²) |
|---|---|---|
| 0.0005% | 2.5 | 12.5 |
| 0.0005% | 10 | 50 |
| 0.004% | 2.5 | 100 |
| 0.004% | 10 | 400 |
| 0.01% | 2.5 | 250 |
| 0.01% | 10 | 1000 |
| 0.05% | 2.5 | 1250 |
| 0.05% | 10 | 5000 |

### Example 17

### Dose Response Study in the Rhino Mouse Model

The rhino mouse is used as a model for the keratinisation defect in acne and has been shown to respond to retinoic acid treatment (J. Invest. Dermatol., 73, 354, (1979); Kligman L.H. and Kligman A.M.). The rhino mouse has abnormal keratin-filled hair follicles (utriculi) and loose sagging skin. On treatment with retinoic acid, the keratin plugs break up and are gradually expelled, and the hair follicle becomes normalised. Other retinoid-induced effects on the skin include thickening of the epidermis and smoothing of the wrinkled skin.

### Method

A dose response study was carried out according to the method of Kligman and Kligman to compare the effects of three different concentration, retinoic acid (tretinoin) supersaturated gel compositions of the invention (0.0005, 0.00125 and 0.0025% w/w tretinoin) against a placebo gel formulation and a subsaturated control (0.005% w/w solution of tretinoin in 70% ethanol (95% w/w)and PEG (5% w/w). Samples (100µl) were applied twice daily, 5 times a week for 4 weeks. 8 animals were treated in each group.

### Assessments

A subjective assessment was made on a weekly basis of wrinkling, roughness (scaling) and irritation (redness) of the skin.
Replicas were made of a standard area of the dorsal skin on 6 mice per group at the beginning and at the end of the study. The replicas were used for measurement of roughness parameters Ra and Rz by Image Analysis. (Ra gives a general measure of roughness and Rz gives a measure of
maximum wrinkle depth, separately from fine wrinkles.)
Split skin whole mounts were prepared from treated skin at the end of the treatment period using 6 mice per group. The epidermis was separated from the dermis and mounted on microscope slides. The keratin-filled follicular structures remained attached to the epidermis (which was almost entirely stratum corneum). The diameters of these utriculi were measured under the microscope.
Sections of skin were prepared for histological evaluation from 6 mice per group. Heamatoxylin and Eosin sections were evaluated for epidermal thickening and inflammatory changes. A global assessment was made of the utriculi changes.

### Results

The results for the subjective assessment of skin condition, together with numerical roughness data derived from the replicas and the whole mount measurements are given in Table I. The same trends are apparent in all three endpoints. All retinoic acid formulations showed biological activity and a dose response, with a numerical trend in all parameters corresponding to the dose applied, was observed. The subsaturated control formulation was the most irritant and produced noticeable flaking of the skin surface. The lowest concentration tested (0.0005% w/w supersaturated gel formulation) was notably less irritant than the other drug formulations tested. It generated only a slight pink colouration of the skin, an indication of minimal irritant effect.

Histological evaluation of the skin sections also demonstrated a clear dose response. Keratinisation defects were observed to be normalised in a dose-dependant manner. Histological evaluation indicated that the biological effects of the 0.0025% w/w supersaturated test formulation and the subsaturated control were similar but inflammation of the dermis (a measure of irritation) in mice treated with the supersaturated formulation was noticeably reduced. Thus, for both formulations the utriculi were greatly narrowed and converted to almost normal follicular dimensions.
All but small amounts of horny material were expelled. The follicular epithelium and the epidermis were hyperplastic (greatly thickened) and the granular layer was prominent.
The lowest dose supersaturated formulation (0.0005% w/w) showed significant changes by comparison with placebo. Epidermal thickening, induction of a granular layer and slight enlargement of the sebaceous glands were observed. Most utriculi were narrowed to shallow "U" shapes with retention of small to moderate amounts of horny material. There was virtually no inflammation of the dermis.

**TABLE I**

| **Sample** | **Clinical Assessment** | **Replicas** mean reduction in roughness ± SD | | | **Whole Mounts** mean diameters (um) ± SD |
|---|---|---|---|---|---|
| | **n = 8** | **n** | **Ra** | **Rz** | **n = 6** |
| Untreated control | - | - | - | - | 10.6 ± 9.2 |
| Placebo gel | No obvious change in the skin | 4 | 3.25 ± 2.71 | 11.28 ± 4.70 | 9.0 ± 0.54 |
| 0.0005% RA supersaturated | Skin slightly pink and a little smoother than normal | 6 | 5.69 ± 5.43 | 20.58 ± 18.04 | 5.5 ± 0.98 |
| 0.00125% RA supersaturated | Skin slightly pink slightly dry (very fine scales) and smoother than normal | 6 | 7.81 ± 3.14 | 26.39 ± 10.50 | 4.5 ± 0.64 |
| 0.0025% RA supersaturated | Skin pink, slightly dry (very fine scales) and smoother than normal | 4 | 7.84 ± 2.78 | 29.55 ± 4.61 | 4.4 ± 0.37 |
| 0.005% RA subsaturated control (EtOH/PEG/H₂O) solution | Skin pink, dry (scales more abundant), and a few mice irritated with tiny red spots | 6 | 2.06 ± 7.31 | 12.87 ± 20.19 | 3.9 ± 0.33 |

## Claims

1. A composition for topical application to the skin comprising a retinoid compound and a vehicle system for the retinoid compound characterised in that the concentration of the retinoid compound is in the range 0.0001 to 0.004% by weight of the composition and the vehicle system is formulated to deliver a supersaturated solution of the retinoid compound to the skin surface; subject to the proviso that when the composition is a two-phase composition calculated to generate a 6-fold supersaturated 0.0025% by weight solution of retinoic acid or a 10-fold supersaturated 0.002% by weight solution of retinyl propionate on mixing of the two phases, then one phase of the composition is not a solution of the retinoid compound in polyethylene glycol, and when the composition is a two-phase composition calculated to generate a 7-fold supersaturated 0.0025% by weight solution of retinoic acid, then one phase of the composition is not a solution of the retinoid compound in propylene glycol when the second phase is a mixture of glycerol, water and ethanol.

2. A composition as claimed in claim 1 in which the retinoid compound is all-trans retinoic acid or 13-cis retinoic acid or an ester or amide derivative thereof; etretinate; retinal; retinol; or a retinol ester.

3. A composition as claimed in claim 2 in which the retinoid compound is all-trans retinoic acid.

4. A composition as claimed in any one of claims 1 to 3 in which the concentration of the retinoid compound is in the range 0.0005 to 0.0025% by weight of the total composition.

5. A composition as claimed in any preceding claim which is a single-phase composition and wherein the vehicle system comprises a mixture of a volatile and a non-volatile solvent.

6. A composition as claimed in claim 5 in which the non-volatile solvent comprises propylene glycol, polyethylene glycol, propylene carbonate, glycerol or mixtures thereof and the volatile solvent comprises ethanol or a mixture of ethanol and water.

7. A composition as claimed in any one of claims 1 to 4 which is a two-phase composition wherein the two phases are intended to be mixed together on or immediately prior to application to the skin, comprising a first liquid phase containing a retinoid compound dissolved therein and comprising a topically acceptable solubiliser; and a second liquid phase, physically and/or chemically different from the first phase but miscible therewith on admixture, optionally containing the same retinoid compound dissolved therein and comprising a topically acceptable carrier; the composition of the first and second liquid phases being such that each has a different lipophilicity and each confers a different saturated solubility on the retinoid compound; the concentration of retinoid compound in each phase in which it is present and the composition of each of the first and second liquid phases being such that, on admixture of the phases the retinoid compound concentration in the mixture thus formed is greater than the saturated retinoid compound concentration in the same mixture, whereby the said mixture is supersaturated with the retinoid.

8. A composition as claimed in claim 7 wherein the topically acceptable carrier of the second liquid phase comprises a first component which is water and a second component which has a lipophilicity intermediate between that of water and the solubiliser of the first liquid phase.

9. A composition as claimed in claim 7 or 8 in which the topically acceptable solubiliser is selected from propylene glycol, 1,3-propylene diol, polyethylene glycol, ethanol, propanol, acetone, dimethylisosorbide, dimethylsulphoxide, benzyl alcohol and other glycol, ether and ester solvents of similar polarity.

10. A composition as claimed in claim 8 or 9 in which the second component of the topically acceptable carrier of the second liquid phase comprises up to 50% by weight of the topically acceptable carrier.

11. A composition as claimed in claim 10 in which the second component of the topically acceptable carrier is glycerol or propylene glycol.

12. A composition as claimed in any one of claims 8 to 11 in which the topically acceptable solubiliser of the first liquid phase comprises a first component which is non-volatile and up to 50% by weight of a relatively more volatile second component which has comparable volatility to water.

13. A composition as claimed in any one of claims 8 to 12 in which the second liquid phase comprises up to 20% by weight of a relatively more volatile second component as defined in claim 12.

14. A composition as claimed in claim 12 or 13 in which the relatively more volatile second component is ethanol, isopropanol or acetone.

15. A composition as claimed in any one of claims 7 to 14 in which the relative proportion by weight of the first liquid phase to the second liquid phase is from 1 : 1 to 1 : 12.

16. A composition as claimed in any one of claims 7 to 15 in which the degree of saturation on admixture of the first and second liquid phases is in the range 2 to 10.

17. A composition as claimed in any preceding claim for use in therapy.

18. The use of a composition as claimed in any one of claims 1 to 16 for the manufacture of a medicament for the treatment or prophylaxis of acne or photoaged or sun-damaged skin.

## Patentansprüche

1. Zusammensetzung zum topischen Auftragen auf die Haut, umfassend eine Retinoidverbindung und ein Trägersystem für die Retinoidverbindung, dadurch gekennzeichnet, daß die Konzentration der Retinoidverbindung im Bereich von 0,0001 bis 0,004 Gew.-%, bezogen auf die Gesamtzusammensetzung, liegt, und daß das Trägersystem so formuliert ist, daß eine übersättigte Lösung der Retinoidverbindung auf die Hautoberfläche übertragen wird, mit der Maßgabe, daß, wenn die Zusammensetzung eine zweiphasige Zusammensetzung ist, für die vorgesehen ist, daß sie nach dem Mischen der beiden Phasen eine sechsfache Übersättigung der 0,0025 Gew.-%igen Lösung der Retinsäure oder eine zehnfache Übersättigung der 0,002 Gew.-%igen Lösung von Retinylpropionat ergibt, eine Phase der Zusammensetzung keine Lösung der Retinoidverbindung in Polyethylenglycol ist, und, wenn die Zusammensetzung eine zweiphasige Zusammensetzung ist, für die vorgesehen ist, daß sie eine siebenfache Übersättigung der 0,0025 Gew.-%igen Lösung der Retinsäure ergibt, eine Phase der Zusammensetzung keine Lösung der Retinoidverbindung in Propylenglycol ist, sofern die zweite Phase ein Gemisch aus Glycerin, Wasser und Ethanol ist.

2. Zusammensetzung nach Anspruch 1, wobei die Retinoidverbindung all-trans Retinsäure oder 13-cis Retinsäure oder ein Ester- oder Amidderivat davon; Etretinat; Retinal; Retinol oder ein Retinolester ist.

3. Zusammensetzung nach Anspruch 2, wobei die Retinoidverbindung all-trans Retinsäure ist.

4. Zusammensetzung nach einem der Ansprüche 1 bis 3, wobei die Konzentration der Retinoidverbindung im Bereich von 0,0005 bis 0,0025 Gew.-%, bezogen auf die Gesamtzusammensetzung, liegt.

5. Zusammensetzung nach einem der vorhergehenden Ansprüche als einphasige Zusammensetzung, wobei das Trägersystem ein Gemisch aus einem flüchtigen und einem nicht flüchtigen Lösungsmittel umfaßt.

6. Zusammensetzung nach Anspruch 5, wobei das nicht flüchtige Lösungsmittel Propylenglycol, Polyethylenglycol, Propylencarbonat, Glycerin oder Gemische davon umfaßt und das flüchtige Lösungsmittel Ethanol oder ein Gemisch aus Ethanol und Wasser umfaßt.

7. Zusammensetzung nach einem der Ansprüche 1 bis 4 als zweiphasige Zusammensetzung, wobei die zwei Phasen bei oder unmittelbar vor dem Auftragen auf die Haut zusammengemischt werden sollen, umfassend eine erste flüssige Phase enthaltend eine darin gelöste Retinoidverbindung und umfassend einen topisch verträglichen Lösungsvermittler, und eine zweite, physikalisch oder chemisch unterschiedliche aber mit der ersten mischbare flüssige Phase, gegebenenfalls mit der gleichen darin gelösten Retinoidverbindung und umfassend einen lokal verträglichen Träger, wobei die Zusammensetzung der ersten und zweiten flüssigen Phasen so ist, daß jede einen anderen Lipophiliegrad aufweist und jede der Retinoidverbindung eine andere Sättigungslöslichkeit verleiht, und wobei die Konzentration der Retinoidverbindung in jeder Phase, in der sie vorliegt, und die Zusammensetzung sowohl der ersten als auch der zweiten flüssigen Phase so ist, daß beim Mischen der Phasen die Konzentration der Retinoidverbindung in dem erhaltenen Gemisch höher ist, als die Sättigungskonzentration der Retinoidverbindung in dem gleichen Gemisch, wobei dieses Gemisch mit dem Retinoid übersättigt ist.

8. Zusammensetzung nach Anspruch 7, wobei der topisch verträgliche Träger der zweiten flüssigen Phase eine erste Komponente umfaßt, die Wasser ist, und eine zweite Komponente umfaßt, die einen Lipophiliegrad aufweist, der zwischen dem von Wasser und dem des Lösungsvermittlers der ersten flüssigen Phase liegt.

9. Zusammensetzung nach Anspruch 7 oder 8, wobei der topisch verträgliche Lösungsvermittler ausgewählt ist aus Propylenglycol, 1,3-Propylendiol, Polyethylenglycol, Ethanol, Propanol, Aceton, Dimethylisosorbid, Dimethylsulfoxid, Benzylalkohol sowie anderen Glycol-, Ether- und Esterlösungsmitteln ähnlicher Polarität.

10. Zusammensetzung nach Anspruch 8 oder 9, wobei die zweite Komponente des topisch verträglichen Trägers der zweiten flüssigen Phase bis zu 50 Gew.-% des lokal verträglichen Trägers ausmacht.

11. Zusammensetzung nach Anspruch 10, wobei die zweite Komponente des topisch verträglichen Trägers Glycerin oder Propylenglycol ist.

12. Zusammensetzung nach einem der Ansprüche 8 bis 11, wobei der topisch verträgliche Lösungsvermittler der ersten flüssigen Phase eine erste nicht flüchtige Komponente und bis zu 50 Gew.-% einer zweiten vergleichsweise flüchtigeren Komponente umfaßt, deren Flüchtigkeit mit der von Wasser vergleichbar ist.

13. Zusammensetzung nach einem der Ansprüche 8 bis 12, wobei die zweite flüssige Phase bis zu 20 Gew.-% einer zweiten vergleichsweise flüchtigeren Komponente, wie in Anspruch 12 definiert, ausmacht.

14. Zusammensetzung nach einem der Ansprüche 12 oder 13, wobei die zweite vergleichsweise flüchtigere Komponente Ethanol, Isopropanol oder Aceton ist.

15. Zusammensetzung nach einem der Ansprüche 7 bis 14, wobei das relative Gewichtsverhältnis der ersten flüssigen Phase zur zweiten flüssigen Phase zwischen 1:1 und 1:12 liegt.

16. Zusammensetzung nach einem der Ansprüche 7 bis 15, wobei der Sättigungsgrad beim Mischen der ersten und zweiten flüssigen Phase im Bereich von 2 bis 10 liegt.

17. Zusammensetzung nach einem der vorstehenden Ansprüche zur therapeutischen Verwendung.

18. Verwendung einer Zusammensetzung rach einem der Ansprüche 1 bis 16 zur Herstellung eines Arzneimittels zur Behandlung oder Vorbeugung von Akne, lichtbedingter Hautalterung oder durch UV-Strahlen hervorgerufener Schädigung der Haut.

## Revendications

1. Composition pour l'application topique sur la peau comprenant un composé rétinoïde et un système véhiculaire pour le composé rétinoïde, caractérisée en ce que la concentration du composé rétinoïde est comprise dans la gamme de 0,0001 à 0,004% en poids de la composition et que le système véhiculaire est formulé pour distribuer une solution sursaturée du composé rétinoïde à la surface de la peau, à condition que lorsque la composition est une composition à deux phases calculée pour générer une solution à 0,0025% en poids sursaturée 6 fois d'acide rétinoïque ou une solution à 0,002% en poids sursaturée 10 fois de propionate de rétinyle lors du mélange des deux phases, alors l'une des phases de la composition n'est pas une solution du composé rétinoïde dans un polyéthylène glycol, et que lorsque la composition est une composition à deux phases calculée pour générer une solution à 0,0025% en poids sursaturée 7 fois d'acide rétinoïque, alors l'une des phases de la composition n'est pas une solution du composé rétinoïde dans du propylène glycol lorsque la seconde phase est un mélange de glycérol, d'eau et d'éthanol.

2. Composition suivant la revendication 1, dans laquelle le composé rétinoïde est l'acide tout trans-rétinoïque ou l'acide 13-cis-rétinoïque ou un dérivé ester ou amide de celui-ci, un étrétinate, le rétinal, le rétinol ou un ester de rétinol.

3. Composition suivant la revendication 2, dans laquelle le composé rétinoïde est l'acide tout trans-rétinoïque.

4. Composition suivant l'une quelconque des revendications 1 à 3, dans laquelle la concentration du composé rétinoïde est comprise dans la gamme de 0,0005 à 0,0025% en poids de la composition totale.

5. Composition suivant l'une quelconque des revendications précédentes, qui est une composition à une seule phase et dans laquelle le système véhiculaire comprend un mélange d'un solvant volatil et d'un solvant non volatil.

6. Composition suivant la revendication 5, dans laquelle le solvant non volatil comprend le propylène glycol, un polyéthylène glycol, le carbonate de propylène, le glycérol ou leurs mélanges, et le solvant volatil comprend l'éthanol ou un mélange d'éthanol et d'eau.

7. Composition suivant l'une quelconque des revendications 1 à 4, qui est une composition à deux phases dans laquelle les deux phases sont conçues pour être mélangées ensemble lors de l'application sur la peau ou immédiatement avant celle-ci, comprenant une première phase liquide dans laquelle est dissous un composé rétinoïde et qui contient un solubilisant acceptable du point de vue topique; et une seconde phase liquide, différente chimiquement et/ou physiquement de la première phase, mais miscible à celle-ci lors du mélange, dans laquelle est éventuellement dissous le même composé rétinoïde et qui contient un support acceptable du point de vue topique; la composition des première et seconde phases liquides étant telle que chacune a une lipophilicité différente et que chacune confère une solubilité à saturation différente au composé rétinoïde, la concentration du composé rétinoïde dans chacune des phases dans laquelle il est présent et la composition de chacune des première et seconde phases liquides étant telles que, lors du mélange des phases, la concentration du composé rétinoïde dans le mélange ainsi formé est supérieure à la concentration à saturation du composé rétinoïde dans le même mélange, si bien que ce mélange est sursaturé en composé rétinoïde.

8. Composition suivant la revendication 7, dans laquelle le support acceptable du point de vue topique de la seconde phase liquide comprend un premier composant qui est l'eau, et un second composant qui a une lipophilicité intermédiaire entre celle de l'eau et du solubilisant de la première phase liquide.

9. Composition suivant les revendications 7 ou 8, dans laquelle le solubilisant acceptable du point de vue topique est choisi parmi le propylène glycol, le 1,3-propylène diol, un polyéthylène glycol, l'éthanol, le propanol, l'acétone, le diméthylisorbide, le diméthylsulfoxyde, l'alcool benzylique et d'autres solvants de type glycol, éther et ester de polarité similaire.

10. Composition suivant les revendications 8 ou 9, dans laquelle le second composant du support acceptable du point de vue topique de la seconde phase liquide comprend jusqu'à 50% en poids du support acceptable du point de vue topique.

11. Composition suivant la revendication 10, dans laquelle le second composant du support acceptable du point de vue topique est le glycérol ou le propylène glycol.

12. Composition suivant l'une quelconque des revendications 8 à 11, dans laquelle le solubilisant acceptable du point de vue topique de la première phase liquide comprend un premier composant qui n'est pas volatil et jusqu'à 50% en poids d'un second composant relativement plus volatil qui a une volatilité comparable à celle de l'eau.

13. Composition suivant l'une quelconque des revendications 8 à 12, dans laquelle la seconde phase liquide comprend jusqu'à 20% en poids d'un second composant relativement plus volatil tel que défini dans la revendication 12.

14. Composition suivant les revendications 12 ou 13, dans laquelle le second composant relativement plus volatil est l'éthanol, l'isopropanol ou l'acétone.

15. Composition suivant l'une quelconque des revendications 7 à 14, dans laquelle la proportion relative en poids de la première phase liquide à la seconde phase liquide est de 1:1 à 1:12.

16. Composition suivant l'une quelconque des revendications 7 à 15, dans laquelle le degré de saturation lors du mélange des première et seconde phase liquides est dans la gamme de 2 à 10.

17. Composition suivant l'une quelconque des revendications précédentes utile en thérapie.

18. Utilisation d'une composition suivant l'une quelconque des revendications 1 à 16, pour la fabrication d'un médicament pour le traitement ou la prophylaxie de l'acné ou d'une peau photovieillie ou endommagée par le soleil.
